(19) Europäisches Patentamt
European Patent Office
Office européen des brevets

(11) Numéro de publication : **0 395 545 B1**

(12) # FASCICULE DE BREVET EUROPEEN

(45) Date de publication du fascicule du brevet : **28.07.93 Bulletin 93/30**

(51) Int. Cl.$^5$ : **C07C 57/13,** C07C 51/12

(21) Numéro de dépôt : **90420203.3**

(22) Date de dépôt : **24.04.90**

(54) **Procédé de préparation d'acides hexènedioiques-1,6.**

(30) Priorité : **28.04.89 FR 8906018**

(43) Date de publication de la demande : **31.10.90 Bulletin 90/44**

(45) Mention de la délivrance du brevet : **28.07.93 Bulletin 93/30**

(84) Etats contractants désignés : **AT BE CH DE DK ES FR GB GR IT LI LU NL SE**

(56) Documents cités :
**EP-A- 0 124 160**
**EP-A- 0 146 859**
**DE-A- 2 303 597**

(73) Titulaire : **RHONE-POULENC CHIMIE**
**25, quai Paul Doumer**
**F-92408 Courbevoie Cédex (FR)**

(72) Inventeur : **Jenck, Jean**
**1, Impasse du Rotagnier**
**F-69680 Chassieu (FR)**
Inventeur : **Deweerdt, Hélène**
**89, Rue Garibaldi**
**F-69006 Lyon (FR)**
Inventeur : **Denis, Philippe**
**16, Allée des Troenes**
**FR-69150 Decines (FR)**
Inventeur : **Kalck, Philippe**
**8, Allée "La Pradine", Auzeville-Tolosane**
**F-31320 Catanet Tolosane (FR)**

(74) Mandataire : **Vignally, Noel et al**
**Rhône-Poulenc Chimie Direction de la Propriété Industrielle Centre de Recherches des Carrières B.P. 62**
**F-69192 Saint-Fons Cédex (FR)**

**EP 0 395 545 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation d'acides hexènedioïques-1,6. Par acide hexènedioïque-1,6 on entend plus particulièrement l'acide hexène-3 dioïque-1,6. L'acide hexène-3 dioïque-1,6 peut être hydrogéné en acide adipique.

L'acide adipique, l'une des matières premières du nylon 66 est produit avec de forts tonnages et de ce seul fait, toute nouvelle voie d'accès à ce diacide et/ou à ses dérivés présente un intérêt de principe immédiatement perceptible.

La présente invention a plus spécifiquement pour objet un procédé de préparation de l'acide hexène-3 dioïque-1,6 par réaction du monoxyde de carbone et de l'eau sur au moins un butène disubstitué par des groupes acyloxy en présence d'un catalyseur à base de palladium.

Il est indiqué dans le brevet américain n° 4,611,082 que la carbonylation d'une solution du diacétoxy-1,4 butène-2 dans un solvant aprotique, polaire et non basique choisi dans le groupe constitué par les nitriles, le bis(méthoxy-2) butène-2, le bis(méthoxy-2 éthyl)éther et le chlorure de méthylène à 80 - 140°C en présence d'un halogénure d'un métal de transition n'est pratiquement pas observée et qu'en présence d'un alcool les vitesses augmentent et sont comparables à celles constatées pour la carbonylation du butène-2 diol-1,4. A propos de ce dernier substrat mentionné il est également indiqué qu'il ne permet pas d'atteindre dans les conditions précitées des rendements satisfaisants en produits linéaires de carbonylation et, dans ce contexte, préférence est donnée aux substrats substitués en position 1,4 par des groupements alcoxy.

Dans ce contexte il apparaît donc que le diacétoxy-1,4 butène-2 ne peut être considéré comme un substrat prometteur pour former des produits linéaires dicarbonylés.

Or le diacétoxy-1,4 butène-2 est aisément accessible par acétoxylation du butadiène. Il serait donc hautement souhaitable de pouvoir disposer d'un procédé permettant d'obtenir avec une efficacité élevée des produits linéaires dicarbonylés à partir du diacétoxy-1,4 butène-2, par exemple, et plus généralement de butènes disubstitués par des groupes acyloxy.

La présente invention a donc pour objet un procédé de préparation d'acides hexènedioïques-1,6 par réaction du monoxyde de carbone et de l'eau sur au moins un butène disubstitué par des groupes acyloxy en présence d'un catalyseur à base de palladium et d'un chlorure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

Il a en effet été trouvé de manière tout à fait surprenante qu'un tel procédé permet de réaliser la dicarbonylation dans des conditions de pression et de température acceptables à l'échelle industrielle, avec une sélectivité appréciable en produit dicarbonylé linéaire.

Le procédé en cause peut être représenté par le schéma réactionnel suivant, lorsqu'on part d'un butène-2 disubstitué en 1,4 par des groupes acyloxy,

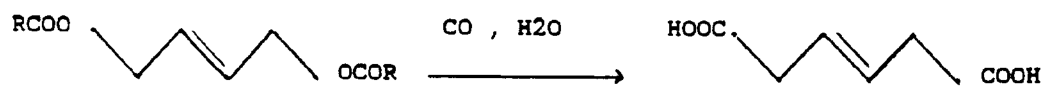


dans lequel R représente :

. un radical alkyle linéaire ou ramifié, contenant de 1 à 12 atomes de carbone, éventuellement substitué par un groupe phényle ;

ou

. un radical aryle contenant de 6 à 10 atomes de carbone, éventuellement substitué par un ou deux radicaux alkyles contenant de 1 à 4 atomes de carbone ;

et peut comporter de 1 à 3 substituants choisis parmi les atomes de fluor et de chlore et les groupements dialkylamino et N,N-dialkylamido dont les radicaux alkyles renferment au plus 4 atomes de carbone.

Le procédé, selon la présente invention, requiert la mise en oeuvre d'au moins un butène disubstitué par des groupes acyloxy. Par groupe acyloxy, on entend des groupes de formule RCOO- dans laquelle R a la signification donnée précédemment ; par butènes disubstitués on entend les composés du butène-2 substitués en positions 1 et 4 et les composés du butène-1 substitués en positions 3 et 4. Bien entendu des mélanges de butène-2 disubstitués par des groupes acyloxy de nature distincte, des mélanges de butène-1 disubstitués par des groupes acyloxy de nature distincte ou des mélanges de butène-2 et de butène-1 disubstitués peuvent être mis en oeuvre dans le cadre du présent procédé.

Il a en effet été constaté par la Demanderesse que la sélectivité en acide linéaire est sensiblement la même que l'on parte d'un butène-2 disubstitué par des groupes acyloxy en positions 1,4 ou d'un butène-1 disubstitué

par des groupes acyloxy en positions 3 et 4.

A titre d'exemples de butènes disubstitués par des groupes acyloxy on peut citer : les diacétoxybutènes, les dipropionyloxybutènes, les dibutyryloxybutènes et les dibenzoyloxybutènes.

Le diacétoxy-1,4 butène-2, le diacétoxy-3,4 butène-1 et leurs mélanges conviennent plus particulièrement bien à la mise en oeuvre de la présente invention.

Le procédé selon la présente invention requiert également la présence d'eau.

La quantité d'eau à mettre en oeuvre dans le cadre du présent procédé n'est pas critique et peut varier dans de larges limites.

Pour une bonne mise en oeuvre de la réaction le rapport molaire de l'eau au butène disubstitué sera compris entre 1 et 100 et, de préférence entre 1 et 50.

Le procédé selon la présente invention est conduit en présence d'un catalyseur à base de palladium.

Bien que la nature précise de l'espèce (ou des espèces) catalytiquement active(s) dans la réaction en cause ne soit pas totalement élucidée, la Demanderesse a constaté que divers composés du palladium et le palladium métallique sont susceptibles d'être des précurseurs utiles dans la mise en oeuvre du présent procédé.

Parmi les sources de palladium pouvant être mises en oeuvre pour réaliser le procédé faisant l'objet de l'invention, on peut citer :

. le palladium métallique déposé le cas échéant sur un support, tel que le charbon, l'alumine, ou la silice,
. $PdCl_2$, $Pd(OAc)_2$, $PBu_4PdCl_3$(Bu = n-butyle)
. les sels ou les complexes $\pi$-allyliques de palladium, dont l'anion coordonné au cation Pd est choisi parmi les anions suivants : carboxylates tels que formiate, acétate, propionate, benzoate ; acétylacétonate, halogénures tels que $Cl^-$ et $Br^-$ et de préférence $Cl^-$ ;

La quantité précise de catalyseur à mettre en oeuvre, qui peut varier dans de larges limites, dépendra avant tout d'un compromis entre l'efficacité souhaitée et la dépense en catalyseur et des autres conditions choisies pour la réaction. En général, de bons résultats peuvent être obtenus avec une concentration de palladium dans le milieu réactionnel, comprise entre $10^{-3}$ et 1 mol/l. De préférence, cette concentration est comprise entre $2.10^{-3}$ et $5.10^{-2}$ Mol/l.

L'une des caractéristiques essentielles du présent procédé réside dans le fait que la réaction est conduite également en présence d'un chlorure d'onium quaternaire d'un élément du groupe VB choisi parmi l'azote et le phosphore, ledit élément étant tétracoordonné à des atomes de carbone, l'azote pouvant en outre être coordonné à deux atomes de phosphore pentavalents.

Par cation onium quaternaire dont l'élément du groupe VB est tétracoordonné à des atomes de carbone, on entend des cations formés à partir d'azote ou de phosphore et de quatre groupements hydrocarbonés monovalents, identiques ou différents, dont la valence libre est portée par un atome de carbone, chaque groupement étant relié à l'élément précité par ladite valence libre, deux quelconques de ces groupements pouvant par ailleurs former ensemble un radical divalent.

Pour une bonne réalisation du procédé de l'invention, le chlorure d'onium quaternaire présente un cation onium quaternaire répondant à l'une des formules I à IV ci-après :

I)

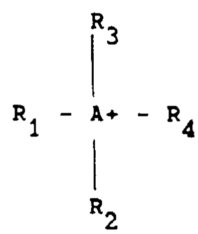

II)

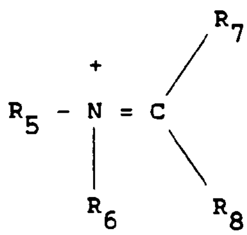

III)

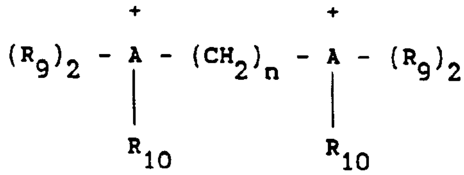

IV)

$$(R_{11})_3 - P = N^+ = P - (R_{11})_3$$

dans lesquelles :

- A représente de l'azote ou du phosphore
- $R_1$, $R_2$,$R_3$, $R_4$ sont identiques ou différents et réprésentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonylé ou halogéno ;
  . deux desdits radicaux $R_1$ à $R_4$ pouvant former ensemble un radical alkylène, alcénylène ou alcadiènylène, linéaire ou ramifié et contenant de 3 à 6 atomes de carbone ;
- $R_5$, $R_6$, $R_7$, $R_5$ sont identiques ou différents et représentent :
  . un radical alkyle linéaire ou ramifié contenant de 1 à 4 atomes de carbone ;
  . les radicaux $R_7$ et $R_5$ pouvant former ensemble un radical alkylène, contenant de 3 à 6 atomes de carbone ;
  . les radicaux $R_6$ et $R_7$ ou $R_5$ et $R_5$ pouvant former ensemble un radical alkylène, alcénylène ou alcadiènylène, contenant 4 atomes de carbone et constituant avec N un hétérocycle azoté ;
- $R_9$ représente un radical alkyle linéaire ou ramifié contenant de 1 à 4 atomes de carbone ou un radical phényle ;
- $R_{10}$ représente :
  . un radical alkyle linéaire ou ramifié contenant de 1 à 4 atomes de carbone, semblable ou différent de $R_9$ ;
  . un radical alcényle linéaire ou ramifié, contenant de 2 à 12 atomes de carbone, de préférence de 4 à 8 atomes de carbone ;
- n représente un nombre entier supérieur ou égal à 1 et inférieur ou égal à 10 et de préférence inférieur ou égal à 6 ;
- $R_{11}$ représente un radical aryle contenant de 6 à 10 atomes de carbone, éventuellement substitué par un ou des groupes alkyle contenant de 1 à 4 atomes de carbone, alcoxy, alcoxycarbonyle ou halogéno.

A titre d'exemple de cations onium quaternaire répondant à la formule I on peut citer les cations :

. tétraméthylammonium,
. triéthylméthylammonium
. tributylméthylammonium
. triméthyl(n-propyl)ammonium
. tétraéthylammonium
. tétrabutylammonium
. dodécyltriméthylammonium
. méthyltrioctylammonium
. heptyltributylammonium
. tétrapropylammonium
. tétrapentylammonium
. tétrahexylammonium
. tétraheptylammonium
. tétraoctylammmonium
. tétradécylammonium
. butyltripropylammonium
. méthyltributylammonium
. pentyltributylammonium

. méthyldiéthylpropylammonium
. éthyldiméthylpropylammonium
. tétradodécylammonium
. tétraoctadécylammonium
. hexadécyltriméthylammonium
. benzyltriméthylammonium
. benzyldiméthylpropylammonium
. benzyldiméthyloctylammonium
. benzyltributylammonium
. benzyltriéthylammonium
. phényltriméthylammonium
. benzyldiméthyltétradécylammonium
. benzyldiméthylhexadécylammonium
. diméthyldiphénylammonium
. méthyltriphénylammonium
. butène-2 yltriéthylammonium
. N,N-diméthyl-tétraméthylènammonium
. N,N-diéthyl-tétraméthylènammonium
. tétraméthylphosphonium
. tétrabutylphosphonium
. éthyltriméthylphosphonium
. triméthylpentylphosphonium
. octyltriméthylphosphonium
. dodécyltriméthylphosphonium
. triméthylphénylphosphonium
. diéthyldiméthylphosphonium
. dicyclohexyldiméthylphosphonium
. diméthyldiphénylphosponium
. cyclohexyltriméthylphosphonium
. triéthylméthylphosphonium
. méthyl-tri(isopropyl)phosphonium
. méthyl-tri(n-propyl)phosphonium
. méthyl-tri(n-butyl)phosphonium
. méthyl-tri(méthyl-2 propyl)phosphonium
. méthyltricyclohexylphosphonium
. méthyltriphénylphosphonium
. méthyltribenzylphosphonium
. méthyl-tri(méthyl-4 phényl)phosphonium
. méthyltrixylylphosphonium
. diéthylméthylphénylphosphonium
. dibenzylméthylphénylphosphonium
. éthyltriphénylphosphonium
. tétraéthylphosphonium
. éthyl-tri(n-propyl)phosphonium
. triéthylpentylphosphonium
. hexadécyltributylphosphonium
. éthyltriphénylphosphonium
. n-butyl-tri(n-propyl)phosphonium
. butyltriphénylphosphonium
. benzyltriphénylphosphonium
. (β-phényléthyl)diméthylphénylphosphonium
. tétraphénylphosphonium
. triphényl(méthyl-4 phényl)phosphonium
. tétrakis(hydroxyméthyl)phosphonium
. tétrakis(hydroxy-2 éthyl)phosphonium

Parmi les cations répondant à la formule II, on peut citer les cations :

. N-méthylpyridinium
. N-éthylpyridinium

. N-hexadécylpyridinium
. N-méthylpicolinium

Parmi les cations répondant à la formule III, on peut citer les cations :

. bis(triméthylammonium)-1,2 éthane
. bis(triméthylammonium)-1,3 propane
. bis(triméthylammonium)-1,4 butane
. bis(triméthylammonium)-1,3 butane

Parmi les cations répondant à la formule IV, on peut citer les cations :

bis(triphénylphosphine)iminium
bis(tritolylphosphine)iminium.

On recourt avantageusement à ceux des cations onium répondant à la formule (I) ci-avant dans laquelle :

- A représente du phosphore et,
- $R_1$, $R_2$, $R_3$ et $R_4$ sont identiques ou différents et représentent un radical alkyle linéaire ou ramifié contenant de 1 à 8 atomes de carbone, un radical phényle ou méthyl-4 phényle.

On utilise de préférence un chlorure de tétralkylphosphonium.

Le chlorure de tétrabutylphosphonium disponible et particulièrement efficace est plus spécialement recommandé.

On notera que certains composés du palladium, tel $PBu_4PdCl_3$ mentionné précédemment et résultant de la réaction entre des quantités molaires équivalentes de $PBu_4Cl$ et de $PdCl_2$, peuvent constituer à la fois une source de palladium et un moyen d'introduction d'un chlorure d'onium quaternaire au sens indiqué ci-avant.

Il a été constaté que l'effet bénéfique apporté par la présence dans le milieu de carbonylation d'un chlorure d'onium quaternaire correspondant à la définition donnée ci-dessus est sensible à partir d'un rapport molaire cation onium/palladium de 0,5 ; notamment un effet particulièrement intéressant a été constaté lorsque ledit rapport est compris entre 1 et 50, un rapport plus élevé pouvant même être choisi sans préjudice pour la réaction. En effet, le chlorure d'onium quaternaire peut être utilisé en quantité relativement importante et jouer en quelque sorte le rôle supplémentaire de diluant du milieu réactionnel.

La réaction pourra être généralement conduite en phase liquide à une température comprise entre 50 et 150°C, de préférence entre 80 et 130°C, sous une pression d'oxyde de carbone comprise entre 10 et 250 bar (1000 et 25 000 KPa), de préférence entre 15 et 180 bar (1500 et 18 000 KPa).

Des gaz inertes, tels l'azote, l'argon ou le gaz carbonique, peuvent être présents à côté de l'oxyde de carbone.

Bien entendu la réaction peut être conduite en présence de solvants ou diluants exogènes au milieu réactionnel tels les hydrocarbures aromatiques, les esters, les cétones, les nitriles ou les amides d'acides carboxyliques.

Selon une variante avantageuse du procédé selon la présente invention la réaction est conduite dans la N-méthylpyrrolidone.

La concentration du butène disubstitué peut varier dans de larges limites.

En fin de réaction ou du temps de réaction voulu, on récupère le diacide recherché par tout moyen approprié, par exemple par extraction.

Les exemples ci-après illustrent l'invention.

Dans ces exemples, le taux de transformation est de 100 % et on observe la formation des divers acides suivants :

- HD : mélange d'acides hexène-3 et hexène-2 dioïques dans lequel l'acide hexène-3 dioïque est majoritaire.
- Ac.$C_5$ : mélange d'acides valérique, méthyl-2 butyrique, pentène-3 oïque, pentène-2 oïque et pentène-4 oïque dans lequel l'acide-pentène-3 oïque est majoritaire.
- $C_6$sat. : mélange d'acides éthylsuccinique, méthylglutarique et adipique dans lequel l'acide méthylglutarique est majoritaire.
- PDO : acide pentadiènoïque

dont on indique pour chaque groupe le nombre de moles formées pour 100 moles de diacétoxybutène chargées.

<u>EXEMPLES 1 A 11 ; Essais témoins (a) et (b) :</u>

Dans un autoclave en acier inoxydable (HastelloyB2) de 125 cm3, préalablement purgé à l'argon, on introduit :

- 8,6 g (50 mmol) de diacétoxy-1,4 butène-2
- 1,8 g (100 mmol) d'eau
- 1 mat-g de palladium sous la forme indiquée dans le tableau I ci-après :

- 5 g (17 mmol) de $PBu_4Cl$
- 25 cm3 de solvant dont la nature figure au tableau I ci-après :

L'autoclave est fermé hermétiquement, placé dans un four agité et raccordé à l'alimentation de gaz sous pression. On purge le réacteur à froid avec de l'oxyde de carbone et on le porte à 100°C. On régule ensuite la pression à 120 bar. Après 6 heures de réaction (sauf indication contraire), l'autoclave est refroidi et dégazé.

Le mélange réactionnel résultant est dilué à 100 cm3 par le solvant.

Une partie aliquote est estérifiée par le méthanol puis analysée par chromatographie en phase gazeuse.

TABLEAU I

| Ex n° | Pd | Solvant | t(h) | Résultats (%) HD | Ac.$C_5$ | C sat. $C_6$ | PDO |
|---|---|---|---|---|---|---|---|
| 1 | $PdCl_2$ | $CH_3CN$ | | 34 | 35 | 17 | 0 |
| (a) | " | " | | 6 | 16 | 5 | 4 |
| (b) | " | " | | 2 | 33 | 11 | 1 |
| 2 | $Pd(OAc)_2$ | " | 2 | 38 | 2 | 1 | 15 |
| 3 * | id+$HBF_4$ | " | | 22 | 32 | 17 | 0 |
| 4 | $PdCl_2$ | NMP | 3 | 68 | 2 | 0 | 12 |
| 5 | $Pd(OAc)_2$ | " | | 22 | 1 | 0 | 41 |
| 6 | $Pd(dba)_2$ | " | | 20 | 0,5 | 0 | 40 |
| 7 | $MePØ_3PdCl_3$ | " | | 60 | 1 | 0 | 12 |
| 8 * | Pd/C | " | | 17 | 3 | - | 54 |
| 9 | $PdCl_2$ | $PBu_4Cl$ | 3 | 80 | 4 | 1 | 1 |
| 10 | " | DMF | | 33 | 0 | 0 | 11 |
| 11 | " | Sulfolane | | 38 | 20 | 0 | 0,5 |

(a) : absence de $PBu_4Cl$

(b) : remplacement de $PBu_4Cl$ par $PBu_4Br$

3 (*) catalyseur : $Pd(OAc)_2$ + 2mmol$HBF_4$

8 (*) catalyseur : Pd/C (10 % en palladium) ; 1 mmol Pd.

t(h) : durée d'absorption si elle est inférieure à 6 heures.

EXEMPLE 12 :

On reproduit l'exemple 4 ci-avant en n'utilisant que 0,5 mat-g de palladium sous forme de $PdCl_2$. En 12 heures de réaction on obtient les résultats suivants :

- HD :80 %
- Ac.$C_5$ : 0,5 %
- $C_6$sat. : 0 %
- PDO : 14 %

EXEMPLE 13 :

On reproduit l'exemple 4 ci-avant en n'utilisant que 0,25 mat-g de palladium sous forme de $PdCl_2$. En 12 heures de réaction on obtient les résultats suivants :

- HD : 65 %
- Ac.$C_5$ : 2 %
- $C_6$sat. : 0 %
- PDO : 12 %

EXEMPLES 14 ET 15 :

On reproduit l'exemple 4 ci-avant en n'utilisant respectivement 2,5 g et 15 g de $PBu_4Cl$ et en ajustant à chaque essai le volume de N-méthylpyrrolidone de telle sorte que le volume total de la charge soit constant.

Les résultats obtenus sont indiqués dans le tableau II ci-après dans lequel on a rappelé les résultats et les conditions particulières des exemples 4 et 9 ci-avant.

TABLEAU II

| | | | Résultats (%) | | | |
|---|---|---|---|---|---|---|
| Ex N° | $PBu_4Cl$(g) | t(h) | HD | Ac.$C_5$ | $C_6$ sat. | PDO |
| 14 | 2,5 | 6 | 55 | 1 | 0 | 13 |
| 4 | 5 | 3 | 68 | 2 | 0 | 12 |
| 15 | 15 | 3 | 80 | 3 | 0 | 3 |
| 9 | 30 | 3 | 78 | 4 | 1 | 1 |

EXEMPLE 16 :

On reproduit l'exemple 4 ci-avant en remplaçant dans la charge le diacétoxy-1,4 butène-2 par une quantité équivalente de diacétoxy-1,2 butène-3.

Après 6 heures de réaction, les résultats obtenus sont les suivants :

- HD : 65 %
- Ac.$C_5$ : 1 %
- $C_6$sat : 0 %
- PDO : 10 %

EXEMPLE 17 :

On reproduit l'exemple 4 ci-avant à 130°C.

Après 3 heures de réaction, les résultats obtenus sont les suivants :

- HD : 45 %

- $Ac.C_5$ : 7 %
- $C_6$sat. : 0 %
- PDO : 0 %

EXEMPLE 18 :

On reproduit l'exemple 4 ci-avant à 70°C.

Après 6 heures de réaction, les résultats obtenus sont les suivants :

- HD : 30 %
- $Ac.C_5$ : 2 %
- $C_6$sat. : 0 %
- PDO : 33 %

EXEMPLE 19 :

On reproduit l'exemple 4 ci-avant sous une pression de 180 bars.

On obtient sensiblement les mêmes résultats.

EXEMPLE 20 :

On reproduit l'exemple 4 ci-avant sous une pression de 15 bars.

Après 6 heures de réaction les résultats obtenus sont les suivants :

- HD : 40 %
- $Ac.C_5$ : 30 %
- $C_6$sat : 0 %
- PDO : 2 %

EXEMPLE 21 :

On reproduit l'exemple 4 ci-avant sous une pression de 60 bars.

Après 6 heures de réaction les résultats obtenus sont les suivants :

- HD : 75 %
- $Ac.C_5$ : 3 %
- $C_6$sat : 0 %
- PDO : 9 %

EXEMPLES 22 A 24 :

On reproduit 2 fois l'exemple 4 ci-avant avec respectivement 25 mmol et 100 mmol de diacétoxy-1,4 butène-2 (DAB) en maintenant dans chaque essai un rapport molaire diacétoxy-1,4 butène-2/$H_2O$ de 1/2 et le volume de la charge constant en jouant sur la quantité de NMP (Exemples 22 et 23).

On reproduit l'exemple 1 ci-avant avec 25 mmol de diacétoxy-1,4 butène-2 en maintenant le rapport molaire de ce dernier avec l'eau à 1/2 et le volume de la charge constant en jouant sur la quantité de $CH_3CN$ (exemple 24).

Les résultats obtenus figurent dans le tableau III ci-après :

TABLEAU III

| Ex n° | Solvant | DAB mmol | t(h) | Résultats (%) HD | $Ac.C_5$ | $C_6$ sat. | PDO |
|---|---|---|---|---|---|---|---|
| 22 | NMP | 25 | 3 | 80 | 1 | ? | 7 |
| 4 | " | 50 | 3 | 68 | 2 | 0 | 12 |
| 23 | " | 100 | | 60 | 2 | 0 | 15 |
| 24 | $CH_3CN$ | 25 | | 68 | 23 | 22 | 0 |
| 1 | " | 50 | | 45 | 35 | 17 | 0 |

t(h) : durée d'absorption si elle est inférieure à 6 heures

EXEMPLES 25 ET 26 :

On reproduit l'exemple 1 ci-avant en faisant varier la quantité d'eau chargée.
Les conditions particulières et les résultats obtenus figurent dans le tableau IV ci-après :

TABLEAU IV

| Ex N° | $H_2O$ mmol | t(h) | Résultats (%) HD | $Ac.C_5$ | $C_6$ sat. | PDO |
|---|---|---|---|---|---|---|
| 1 | 100 | 6 | 45 | 35 | 17 | 0 |
| 25 | 200 | 2,5 | 48 | 27 | 9 | 0 |
| 26 | 500 | 3 | 45 | 4 | 5 | 5 |

## Revendications

1. Procédé de préparation d'acides hexènedioïques-1,6 par réaction de l'oxyde de carbone et de l'eau sur au moins un butène disubstitué par des groupes acyloxy en présence d'un catalyseur à base de palladium et d'au moins un chlorure d'onium quaternaire d'un élément du groupe VB choisi parmi l'azote et le phosphore, ledit élément étant tétracoordonné à des atomes de carbone, l'azote pouvant être coordonné à deux atomes de phosphore pentavalents.

2. Procédé selon la revendication 1, caractérisé en ce que le chlorure d'onium quaternaire d'un élément du groupe VB choisi parmi l'azote et le phosphore présente un cation onium quaternaire répondant à l'une des formules (I) à (IV) suivantes :

I)

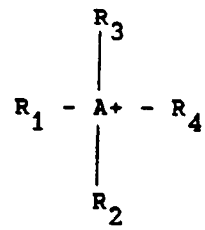

II)

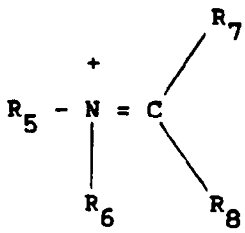

III)

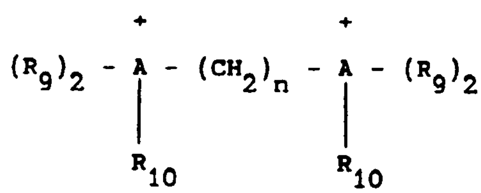

(IV)

$$(R_{11})_3 - P = N^+ = P - (R_{11})_3$$

Dans lesquelles :

- A représente de l'azote ou du phosphore
- $R_1$, $R_2$, $R_3$, $R_4$ sont identiques ou différents et réprésentent :
  . un radical alkyle linéaire ou ramifié contenant de 1 à 16 atomes de carbone, éventuellement substitué par un groupe phényle, hydroxy, halogéno, nitro, alcoxy ou alcoxycarbonyle ;
  . un radical alcényle linéaire ou ramifié contenant de 2 à 12 atomes de carbone, de préférence de 4 à 8 atomes de carbone,
  . un radical aryle contenant de 6 à 10 atomes de carbone, éventuellement substitué par un ou des radicaux alkyles contenant de 1 à 4 atomes de carbone, alcoxy, alcoxycarbonylé ou halogéno ;
  . deux desdits radicaux $R_1$ à $R_4$ pouvant former ensemble un radical alkylène, alcénylène ou alcadiènylène, linéaire ou ramifié et contenant de 3 à 6 atomes de carbone ;
- $R_5$, $R_5$, $R_7$, $R_8$ sont identiques ou différents et représentent :
  . un radical alkyle linéaire ou ramifié contenant de 1 à 4 atomes de carbone;

. les radicaux $R_7$ et $R_8$ pouvant former ensemble un radical alkylène, contenant de 3 à 6 atomes de carbone ;
. les radicaux $R_6$ et $R_7$ ou $R_6$ et $R_8$ pouvant former ensemble un radical alkylène, alcénylène ou alcadiènylène, contenant 4 atomes de carbone et constituant avec N un hétérocycle azoté ;

- $R_9$ représente un radical alkyle linéaire ou ramifié contenant de 1 à 4 atomes de carbone ou un radical phényle ;
- $R_{10}$ représente :
  . un radical alkyle linéaire ou ramifié contenant de 1 à 4 atomes de carbone, semblable ou différent de $R_9$ ;
  . un radical alcényle linéaire ou ramifié, contenant de 2 à 12 atomes de carbone, de préférence de 4 à 8 atomes de carbone ;
- n représente un nombre entier supérieur ou égal à 1 et inférieur ou égal à 10 et de préférence inférieur ou égal à 6 ;
- $R_{11}$ représente un radical aryle contenant de 6 à 10 atomes de carbone, éventuellement substitué par un ou des groupes alkyle contenant de 1 à 4 atomes de carbone, alcoxy, alcoxycarbonylé ou halogéno.

**3.** Procédé selon la revendication 1 ou 2, caractérisé en ce que le cation onium quaternaire répond à la formule (I) donnée dans la revendication 2, dans laquelle :

- A représente du phosphore et,
- $R_1$, $R_2$, $R_3$ et $R_4$ sont identiques ou différents et représentent un radical alkyle linéaire ou ramifié contenant de 1 à 8 atomes de carbone, un radical phényle ou méthyl-4 phényle.

**4.** Procédé selon l'une quelconque des revendications précédentes, caractérisé en ce que le chlorure d'onium quaternaire est le chlorure de tétrabutylphosphonium.

**5.** Procédé selon l'une quelconque des revendications précédentes, caractérisé en ce que le rapport molaire du cation onium au palladium est supérieur ou égal à 1.

**6.** Procédé selon l'une quelconque des revendications précédentes, caractérisé en ce que la concentration du palladium dans le milieu réactionnel est comprise entre $10^{-3}$ et 1 mol/l.

**7.** Procédé selon l'une quelconque des revendications précédentes, caractérisé en ce que le rapport molaire de l'eau au butène disubstitué est compris entre 1 et 100 et, de préférence, entre 1 et 50.

**8.** Procédé selon l'une quelconque des revendications précédentes, caractérisé en ce que la température de réaction est comprise entre 50 et 150°C et, de préférence, entre 80 et 130°C.

**9.** Procédé selon l'une quelconque des revendications précédentes, caractérisé en ce que la pression est comprise entre 10 et 250 bar (1000 et 25 000 KPa) et, de préférence, entre 15 et 180 bar (1500 et 18 000 KPa).

**10.** Procédé selon l'une quelconque des revendications précédentes, caractérisé en ce que le butène disubstitué est choisi parmi le diacétoxy-1,4 butène-2 et le diacétoxy-3,4 butène-1 et leurs mélanges.

**11.** Procédé selon l'une quelconque des revendications précédentes, caractérisé en ce que la réaction est conduite en présence d'un solvant ou diluant organique.

**12.** Procédé selon la revendication 11, caractérisé en ce que le solvant est la N-méthylpyrrolidone.

## Patentansprüche

**1.** Verfahren zur Herstellung von Hexen-1,6-disäuren durch Umsetzung von Kohlenmonoxid und Wasser mit zumindest einem durch Acyloxygruppen disubstituierten Buten in Gegenwart eines Katalysators auf Basis von Palladium und zumindest einem quaternären Oniumchlorid eines Elements der Gruppe VB, ausgewählt unter Stickstoff und Phosphor, wobei dieses Element mit Kohlenstoffatomen tetrakoordiniert ist, wobei der Stickstoff mit zwei fünfwertigen Phosphoratomen koordiniert sein kann.

**2.** Verfahren gemäß Anspruch 1, dadurch gekennzeichnet, daß das quaternäre Oniumchlorid eines Elements der Gruppe VB, ausgewählt unter Stickstoff und Phosphor, ein quaternäres Oniumkation einer der folgenden Formeln (I) bis (IV) darstellt:

I)

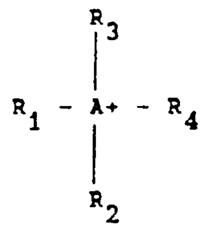

II)

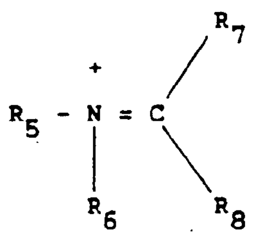

III)

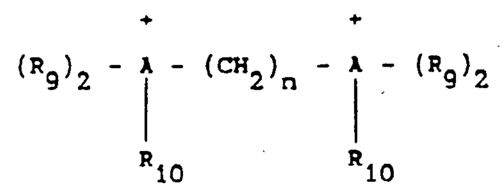

IV)

$$(R_{11})_3 - P = N^+ = P - (R_{11})_3$$

worin:

- A für den Stickstoff oder den Phosphor steht,
- $R_1$, $R_2$, $R_3$, $R_4$ identisch oder verschieden sind und bedeuten:
  . einen linearen oder verzweigten Alkylrest mit 1 bis 16 Kohlenstoffatomen, der gegebenenfalls durch eine Phenyl-, Hydroxy-, Halogen-, Nitro-, Alkoxy- oder Alkoxycarbonylgruppe substituiert ist;
  . einen linearen oder verzweigten Alkenylrest mit 2 bis 12 Kohlenstoffatomen, vorzugsweise 4 bis 8 Kohlenstoffatomen,
  . einen Arylrest mit 6 bis 10 Kohlenstoffatomen, der gegebenenfalls durch einen oder mehrere Alkylreste mit 1 bis 4 Kohlenstoffatomen, Alkoxy, Alkoxycarbonyl oder Halogen substituiert ist,
  . wobei zwei dieser Reste $R_1$ bis $R_4$ gemeinsam einen linearen oder verzweigten Alkylen-, Alkenylen- oder Alkadienylenrest mit 3 bis 6 Kohlenstoffatomen bilden können,
- $R_5$, $R_5$, $R_7$, $R_8$ identisch oder voneinander verschieden sind und bedeuten:
  . einen linearen oder verzweigten Alkylrest mit 1 bis 4 Kohlenstoffatomen,
  . wobei die Reste $R_7$ und $R_8$ gemeinsam einen Alkylenrest mit 3 bis 6 Kohlenstoffatomen bilden können,
  . wobei die Reste $R_5$ und $R_7$ oder $R_8$ und $R_8$ gemeinsam einen Alkylen-, Alkenylen- oder Alkadienylenrest bilden können, der 4 Kohlenstoffatome enthält und mit N einen Stickstoffheterocyclus bildet,
- $R_8$ einen linearen oder verzweigten Alkylrest mit mit 1 bis 4 Kohlenstoffatomen oder einen Phenylrest bedeutet,
- $R_{10}$ bedeutet:
  . einen linearen oder verzweigten Alkylrest mit 1 bis 4 Kohlenstoffatomen, entsprechend $R_9$ oder von $R_9$ verschieden,
  . einen linearen oder verzweigten Alkenylrest mit 2 bis 12 Kohlenstoffatomen, vorzugsweise 4 bis 8 Kohlenstoffatomen,
- n eine ganze Zahl von höher als oder gleich 1 und niedriger als oder gleich 10 und vorzugsweise niedriger als oder gleich 6 bedeutet,
- $R_{11}$ einen Arylrest mit 6 bis 10 Kohlenstoffatomen, gegebenenfalls substituiert durch eine oder mehrere Alkylgruppen mit 1 bis 4 Kohlenstoffatomen, Alkoxy, Alkoxycarbonyl oder Halogen bedeutet.

3. Verfahren gemäß Anspruch 1 oder 2, dadurch gekennzeichnet, daß das quaternäre Oniumkation der Formel (I) gemäß Anspruch 2 entspricht, worin
   - A für Phosphor steht und
   - $R_1$, $R_2$, $R_3$ und $R_4$ identisch oder voneinander verschieden sind und einen linearen oder verzweigten Alkylrest mit 1 bis 8 Kohlenstoffatomen, einen Phenylrest oder einen 4-Methylphenylrest bedeuten.

4. Verfahren gemäß einem der vorhergehenden Ansprüche, dadurch gekennzeichnet, daß das quaternäre Oniumchlorid das Tetrabutylphosphoniumchlorid ist.

5. Verfahren gemäß einem der vorhergehenden Ansprüche, dadurch gekennzeichnet, daß das Molverhältnis des Oniumkations zu dem Palladium höher als oder gleich 1 beträgt.

6. Verfahren gemäß einem der vorhergehenden Ansprüche, dadurch gekennzeichnet, daß die Konzentration des Palladiums in dem Reaktionsmilieu zwischen $10^{-3}$ und 1 Mol/l liegt.

7. Verfahren gemäß einem der vorhergehenden Ansprüche, dadurch gekennzeichnet, daß das Molverhältnis von Wasser zu disubstituiertem Buten zwischen 1 und 100 und vorzugsweise zwischen 1 und 50 beträgt.

8. Verfahren gemäß einem der vorhergehenden Ansprüche, dadurch gekennzeichnet, daß die Reaktionstemperatur zwischen 50 und 150°C und vorzugsweise zwischen 80 und 130°C beträgt.

9. Verfahren gemäß einem der vorhergehenden Ansprüche, dadurch gekennzeichnet, daß der Druck zwischen 10 und 250 bar (1 000 und 25 000 kPa) und vorzugsweise zwischen 15 und 180 bar (1 500 und 18 000 kPa) beträgt.

10. Verfahren gemäß einem der vorhergehenden Ansprüche, dadurch gekennzeichnet, daß das disubstituierte Buten unter 1,4-Diacetoxybut-2-en und 3,4-Diacetoxybut-1-en und deren Mischungen ausgewählt wird.

11. Verfahren gemäß einem der vorhergehenden Ansprüche, dadurch gekennzeichnet, daß die Umsetzung in Gegenwart eines organischen Lösungs- oder Verdünnungsmittels durchgeführt wird.

12. Verfahren gemäß Anspruch 11, dadurch gekennzeichnet, daß das Lösungsmittel N-Methylpyrrolidon ist.

## Claims

1. Process for the preparation of hexene-1,6-dioic acids by reaction of carbon monoxide and water on at least one butene disubstituted by acyloxy groups in the presence of a catalyst based on palladium and at least one quaternary onium chloride of a group VB element chosen from nitrogen and phosphorus, the said element being tetracoordinated to carbon atoms and it being possible for nitrogen to be coordinated to two pentavalent phosphorus atoms.

2. Process according to claim 1, characterized in that the quaternary onium chloride of a group VB element chosen from nitrogen and phosphorus has a quaternary onium cation corresponding to one of the formulae (I) to (IV) below:
   I)

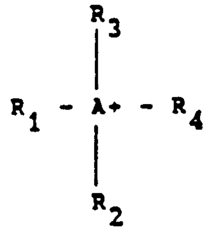

II)

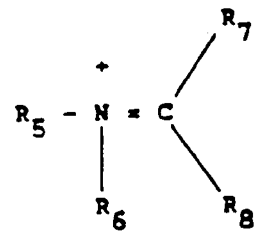

III)

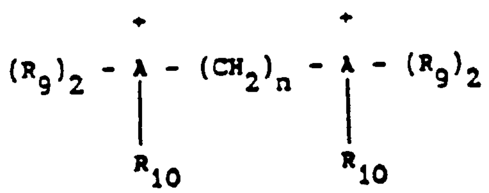

IV)

$$(R_{11})_3 - P = N^+ = P - (R_{11})_3$$

in which formulae:

- A represents nitrogen or phosphorus
- $R_1$, $R_2$, $R_3$ and $R_4$ are identical or different and represent:
  - . a straight-chain or branched alkyl radical containing 1 to 16 carbon atoms, optionally substituted by a phenyl, hydroxyl, halogeno, nitro, alkoxy or alkoxycarbonyl group;
  - . a straight-chain or branched alkenyl radical containing 2 to 12 carbon atoms, preferably 4 to 8 carbon atoms,
  - . an aryl radical containing 6 to 10 carbon atoms, optionally substituted by one or more alkyl radicals containing 1 to 4 carbon atoms, alkoxy, alkoxycarbonyl or halogeno;
  - . it being possible for two of the said radicals $R_1$ to $R_4$ to form together a straight-chain or branched alkylene, alkenylene or alkadienylene radical containing 3 to 6 carbon atoms;
- $R_5$, $R_5$, $R_7$ and $R_8$ are identical or different and represent:
  - . a straight-chain or branched alkyl radical containing 1 to 4 carbon atoms;
  - . it being possible for the radicals $R_7$ and $R_8$ to form together an alkylene radical containing 3 to 6 carbon atoms;
  - . it being possible for the radicals $R_6$ and $R_7$ or $R_6$ and $R_5$ to form together an alkylene, alkenylene or alkadienylene radical containing 4 carbon atoms and forming, with N, a nitrogen-containing heterocyclic radical;
- $R_9$ represents a straight-chain or branched alkyl radical containing 1 to 4 carbon atoms or a phenyl radical;
- $R_{10}$ represents:
  - . a straight-chain or branched alkyl radical containing 1 to 4 carbon atoms, similar to or different from $R_9$;
  - . a straight-chain or branched alkenyl radical containing 2 to 12 carbon atoms, preferably 4 to 8 carbon atoms;
- n represents an integer greater than or equal to 1 and less than or equal to 10 and preferably less than or equal to 6;
- $R_{11}$ represents an aryl radical containing 6 to 10 carbon atoms, optionally substituted by one or more alkyl groups containing 1 to 4 carbon atoms, alkoxy, alkoxycarbonyl or halogeno.

**3.** Process according to claim 1 or 2, characterized in that the quaternary onium cation corresponds to the formula (I) given in claim 2, in which:

- A represents phosphorus, and
- $R_1$, $R_2$, $R_3$ and $R_4$ are identical or different and represent a straight-chain or branched alkyl radical containing 1 to 8 carbon atoms or a phenyl or 4-methylphenyl radical.

**4.** Process according to any one of the preceding claims, characterized in that the quaternary onium chloride is tetrabutylphosphonium chloride.

**5.** Process according to any one of the preceding claims, characterized in that the molar ratio of the onium cation to palladium is greater than or equal to 1.

**6.** Process according to any one of the preceding claims, characterized in that the concentration of palladium in the reaction mixture is between $10^{-3}$ and 1 mol/l.

**7.** Process according to any one of the preceding claims, characterized in that the molar ratio of water to disubstituted butene is between 1 and 100 and preferably between 1 and 50.

**8.** Process according to any one of the preceding claims, characterized in that the reaction temperature is between 50 and 150°C and preferably between 80 and 130°C.

**9.** Process according to any one of the preceding claims, characterized in that the pressure is between 10 and 250 bar (1000 and 25,000 KPa) and preferably between 15 and 180 bar (1500 and 18,000 KPa).

**10.** Process according to any one of the preceding claims, characterized in that the disubstituted butene is chosen from 1,4-diacetoxy-but-2-ene and 3,4-diacetoxybut-1-ene and their mixtures.

**11.** Process according to any one of the preceding claims, characterized in that the reaction is carried out in the presence of an organic solvent or diluent.

**12.** Process according to claim 11, characterized in that the solvent is N-methylpyrrolidone.